# EUROPÄISCHE PATENTSCHRIFT

(11) **EP 3 519 377 B1**
(45) Veröffentlichungstag und Bekanntmachung des Hinweises auf die Patenterteilung: **20.05.2020**
(21) Anmeldenummer: 17818182.2
(22) Anmeldetag: 22.12.2017
(51) Int. Cl.: C07C 5/48, C07C 51/215, C07C 11/04, C07C 53/08

(54) **VERFAHREN UND ANLAGE ZUR HERSTELLUNG VON ETHYLEN UND ESSIGSÄURE**
METHOD AND INSTALLATION FOR THE PRODUCTION OF ETHYLENE AND ACETIC ACID
PROCÉDÉ ET INSTALLATION DE PRODUCTION D'ÉTHYLÈNE ET ACIDE ACÉTIQUE

(30) Priorität: 22.12.2016 EP 16206435
(43) Veröffentlichungstag der Anmeldung: 07.08.2019
(73) Patentinhaber: Linde Aktiengesellschaft, 80331 München (DE)
(72) Erfinder: ZELLHUBER, Mathieu, 82152 Martinsried (DE); WINKLER, Florian, 81241 München (DE); SCHUBERT, Martin, 81375 München (DE); MEISWINKEL, Andreas, 83253 Rimsting (DE)
(86) Internationale Anmeldenummer: PCT/EP2017/084338
(87) Internationale Veröffentlichungsnummer: WO 2018/115416

(56) Entgegenhaltungen:
- EP-A2- 1 201 630
- US-A- 4 899 003

## Beschreibung

Die Erfindung betrifft ein Verfahren zur Herstellung von Ethylen und Essigsäure und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den jeweiligen Olefinen und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und López Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Bei der ODH werden insbesondere bei Verwendung von MoVNbTeOx-Katalysatoren unter industriell relevanten Reaktionsbedingungen signifikante Mengen der jeweiligen Carbonsäuren der eingesetzten Paraffine als Nebenprodukte gebildet. Für einen wirtschaftlichen Anlagenbetrieb ist eine entsprechende Koppelproduktion von Olefinen und der jeweiligen Carbonsäuren bei Verwendung des beschriebenen Katalysatortyps in der Regel erforderlich. Dies gilt insbesondere für die Herstellung von Ethylen durch ODH von Ethan (ODH-E), bei der gleichzeitig Essigsäure gebildet wird.

In der industriellen Praxis gelten Koppelproduktionsverfahren als weniger interessant, da sie immer mit einer begrenzten Produktionsflexibilität einhergehen. Um ein solches Verfahren attraktiv zu gestalten, muss dem Betreiber eine einfach steuerbare, flexible Anlage zur Verfügung gestellt werden, um eine möglichst einfache Anpassung der Produktverteilung an den tatsächlichen und/oder wirtschaftlich sinnvollen Bedarf zu ermöglichen. Die vorliegende Erfindung stellt sich diese Aufgabe.

### Offenbarung der Erfindung

Vor diesem Hintergrund schlägt die vorliegende Erfindung ein Verfahren zur Herstellung von Ethylen und Essigsäure und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Stoffströme, Gasgemische usw. können im hier verwendeten Sprachgebrauch reich oder arm an einer oder mehreren Komponenten sein, wobei die Angabe "reich" für einen Gehalt von wenigstens 95%, 96%, 97%, 98%, 99%, 99,5%, 99,9% oder 99,99% und die Angabe "arm" für einen Gehalt von höchstens 5%, 4%, 3%, 2%, 1%, 0,5%, 0,1% oder 0,01% auf molarer, Gewichts- oder Volumenbasis stehen kann. Sind mehrere Komponenten angegeben, bezieht sich die Angabe "reich" oder "arm" auf die Summe aller Komponenten. Ist hier beispielsweise von "Sauerstoff" oder "Ethan" die Rede, kann es sich um ein Reingas, aber auch um ein an der jeweiligen Komponente reiches Gemisch handeln.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck-bzw. Temperaturwerte verwendet werden müssen. Ein Druckniveau oder Temperaturniveau kann beispielsweise ± 1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

Bei einer "Rektifikationskolonne" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, eingespeistes Stoffgemisch durch Rektifikation zumindest teilweise aufzutrennen, also aus dem Stoffgemisch jeweils Reinstoffe oder zumindest Stoffgemische mit anderer Zusammensetzung zu erzeugen. Typischerweise sind Rektifikationskolonnen als zylindrische Metallbehälter ausgebildet, die mit Einbauten, beispielsweise Trennböden oder geordneten oder ungeordneten Packungen, ausgerüstet sind. Eine Rektifikationskolonne weist einen Sumpfverdampfer auf. Hierbei handelt es sich um eine Einrichtung mit einem Wärmetauscher, der beheizt wird, und der dafür eingerichtet ist, eine im Sumpf der Rektifikationskolonne anfallende flüssige Fraktion, auch als Sumpfflüssigkeit bezeichnet, zu erwärmen. Mittels eines Sumpfverdampfers wird kontinuierlich ein Teil des Sumpfprodukts verdampft und gasförmig in den Trennbereich zurückgespeist.

### Vorteile der Erfindung

Wie erwähnt, ist für einen wirtschaftlichen Anlagenbetrieb eine Koppelproduktion von Ethylen und Essigsäure bei Verwendung des beschriebenen Katalysatortyps in der ODH-E in der Regel erforderlich, obwohl in der industriellen Praxis Koppelproduktionsverfahren als weniger attraktiv gelten. Die Ausgestaltung eines flexiblen, katalytischen Prozesses ist herausfordernd, insbesondere wenn es sich um einen exothermen Prozess wie die ODH-E handelt. In diesem Fall muss stets die Gefahr eines thermischen Durchgangs unterbunden werden, was die Einstellung der Betriebsparameter teilweise stark einschränkt. Des Weiteren beinhalten die katalytischen Prozesse eine Vielzahl von Teilreaktionen, die sich wechselseitig beeinflussen. Es ist daher in der Regel sehr schwierig, geeignete Prozessgrößen zu identifizieren, die die Reaktion zuverlässig beschreiben und als Prozessleitgröße geeignet sind.

Ist nachfolgend von einer Produktion von Ethylen und Essigsäure die Rede, schließt dies nicht aus, dass im Rahmen des erfindungsgemäßen Verfahrens auch höhere Olefine und Carbonsäuren gebildet werden, insbesondere bei Verwendung von entsprechenden Einsätzen, die höhere Paraffine enthalten. Während beispielsweise beim Steamcracken auch aus schwereren Paraffinen leichtere Olefine gebildet werden können, beispielsweise aus Propan Ethylen, ist dies bei der ODH, insbesondere der ODH-E, typischerweise nicht der Fall. So wird Propan hier vorwiegend zu Propylen und Acrylsäure (Propensäure), nicht aber zu Ethylen umgesetzt. Die in der ODH gebildeten Carbonsäuren werden typischerweise mit Wasser aus einem in der ODH gebildeten Prozessgasstrom abgetrennt. Werden Paraffine unterschiedlicher Kettenlängen eingesetzt, erhält man dabei eine wässrige Lösung unterschiedlicher Carbonsäuren. Ist dies, und die gleichzeitige Bildung höherer Olefine, nicht erwünscht, kann ein Reaktionseinsatzstrom derart gebildet werden, dass er keine höheren Paraffine enthält, beispielsweise durch eine stromauf vorgesehene Abtrennung.

Die vorliegende Erfindung beruht auf der überraschenden Erkenntnis, dass bei einem Wasserpartialdruck am Austritt eines oder mehrerer für die ODH-E verwendeter Reaktoren im Bereich von 0,5 bis 5 bar (abs.), insbesondere von 0,7 bis 3 bar (abs.), das Molenstromverhältnis von Essigsäure zu Ethylen im Austrittsstrom (nachfolgend überwiegend als "Prozessgas" bezeichnet) beinahe linear zum Wasserpartialdruck am Austritt verläuft. Dieser Wert lässt sich daher als Prozessleitgröße verwenden, wenn ein bestimmtes Produktverhältnis von Essigsäure zu Ethylen eingestellt werden soll. Der Wasserpartialdruck im Prozessgas ist dabei das Ergebnis sowohl der Wasserzugabe am Reaktoreintritt bzw. in einem entsprechenden Reaktionseinsatzstrom als auch der Umsetzung des Ethans im Reaktor und damit ggf. auch der momentanen Katalysatoraktivität. Im Gegensatz zu einer Einstellung lediglich des Wassergehalts im Reaktionseinsatzstrom, der ohne Kenntnis der genannten weiteren Einflussgrößen zu stark schwankenden Wasserpartialdrücken im Prozessgas und damit schwankenden Produktverhältnissen führen kann, kann durch die Verwendung des Wasserpartialdrucks im Prozessgas als Prozessleitgröße daher eine sehr viel exaktere Einstellung des gewünschten Produktverhältnisses erzielt werden.

Eine Einstellung des Wassergehalts im Reaktionseinsatzstrom, nicht aber im Prozessgas, ist in der EP 1 201 630 A2 beschrieben. Ferner ist hier auch angegeben, dass Druck, Temperatur und Verweildauer in der Reaktionszone kontrolliert werden können. Wie hoch der Wassergehalt im Prozessgas ist, ist allerdings hier nicht thematisiert. Entsprechendes gilt auch für ein in der US 4,899,003 A beschriebenes Verfahren. Es fehlt damit in beiden Fällen an der erfindungsgemäßen Erkenntnis, dass der Wasserpartialdruck am Reaktoraustritt eine Prozessleitgröße darstellt, über die sich die Produktselektivität eines Verfahrens, in dem eine Koppelproduktion von Ethylen und Essigsäure mittels ODH-E unter Verwendung des erwähnten Katalysatortyps vorgenommen wird, besonders zuverlässig einstellen lässt.

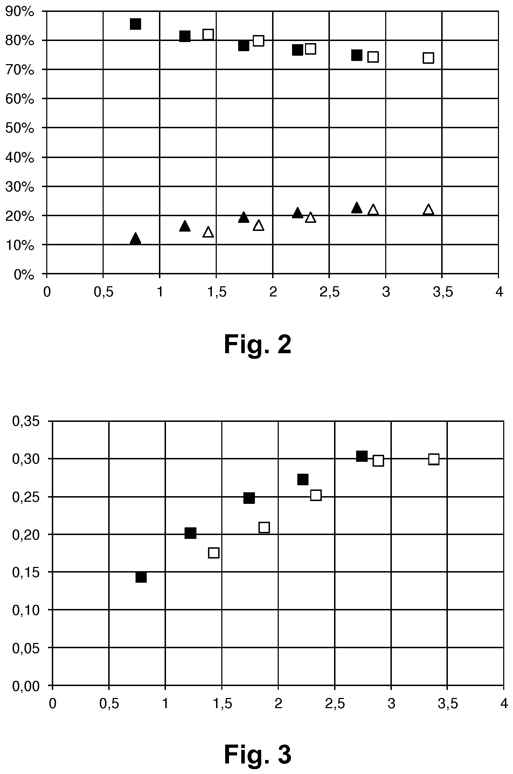

Die genannten, erfindungsgemäßen Gesetzmäßigkeiten wurden zunächst im Rahmen von Ethanoxidationsversuchsreihen mit konstanter Eintrittstemperatur und variierendem Wasseranteil im Reaktionseinsatzstrom unter Verwendung eines MoVNbTeOx-Katalysators festgestellt. Dabei konnte eine nahezu konstante Konversion des Ethans erreicht werden, bei ebenfalls nahezu konstanter Selektivität zu Kohlendioxid und Kohlenmonoxid. Die molaren Mengen an den gewünschten Produkten Ethylen und Essigsäure entwickelten sich hingegen in genau diesem Bereich konträr gegeneinander. In dem genannten Bereich zeigt sich ein stetiger, beinahe linearer gegensätzlicher Verlauf des Produktmolenstromverhältnisses von Essigsäure zu Ethylen. Zur weiteren Erläuterung wird auf die beigefügten Figuren 2 und 3 und die zugehörigen Erläuterungen verwiesen.

Es wurden zusätzlich analoge Versuchsreihen bei unterschiedlichen Durchströmraten und somit unterschiedlicher Raumgeschwindigkeit (Weight Hourly Space Velocity, WHSV) und Temperatur im Reaktor durchgeführt. Bei höherer Durchströmrate und somit höherer Raumgeschwindigkeit und niedrigerer Temperatur sind erwartungsgemäß niedrigere Konversionsraten zu beobachten, jedoch ist das Verhältnis der beiden Produktmolenströme bei gleichen Wasserpartialdrücken am Reaktoraustritt nahezu identisch mit den bei niedrigerer Durchströmrate bestimmten Werten. Dies zeigt, dass die Prozesssteuerung in dem genannten Bereich maßgeblich auf den Wasserpartialdruck am Austritt gestützt werden kann. Der teilweise klar lineare Verlauf des Produktmolenstromverhältnisses wird vor allem für den wirtschaftlich relevanten Betrieb bei höheren Konversionen ersichtlich.

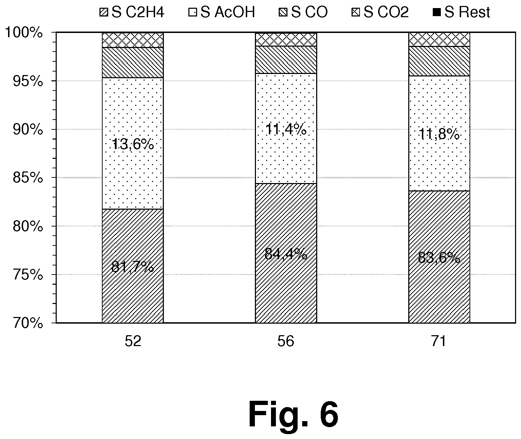

Weitere Versuchsreihen wurden unter Verwendung eines Versuchsreaktors durchgeführt, wobei ebenfalls die oben erwähnten Zusammenhänge belegt werden konnten. Zu Details sei insbesondere auf die beigefügte Figur 6 sowie die zugehörigen Erläuterungen verwiesen.

Die vorliegende Erfindung schlägt daher ein Verfahren zur Herstellung von Ethylen und Essigsäure vor, bei dem ein Ethan und Sauerstoff enthaltender Reaktionseinsatzstrom gebildet und ein Teil des Ethans und des Sauerstoffs in dem Reaktionseinsatzstrom unter Erhalt eines Prozessgases durch oxidative Dehydrierung zu dem Ethylen und der Essigsäure umgesetzt wird, wobei das Prozessgas den nicht umgesetzten Teil des Ethans und des Sauerstoffs, das Ethylen und die Essigsäure sowie Wasser enthält. Wie erwähnt, enthält ein entsprechendes Prozessgas auch weitere Nebenprodukte wie Kohlenmonoxid und Kohlendioxid. Erfindungsgemäß ist vorgesehen, dass in dem Prozessgas ein Wasserpartialdruck in Abhängigkeit von einem vorgegebenen Produktverhältnis, insbesondere einem vorgegebenen Produktmolenstromverhältnis, von der Essigsäure zu dem Ethylen auf einen Wert in einem Bereich zwischen 0,5 bis 5 bar (abs.), insbesondere in einem Bereich zwischen 0,7 und 3 bar (abs.), eingestellt wird. Wie erwähnt, ergibt sich in dem Bereich für unterschiedliche Konversionen und Betriebsbedingungen ein gleichbleibend stetiges, nahezu lineares Produktmolenstromverhältnis von Essigsäure zu Ethylen, so dass hier eine besonders gut steuerbare Koppelproduktion dieser Verbindungen mit einstellbarem Produktionsschwerpunkt möglich ist.

Ein "Reaktionseinsatzstrom" ist im hier verwendeten Sprachgebrauch das gesamte, der ODH unterworfene Gasgemisch. Dieses kann insbesondere auch in Form separater Stoffströme dem oder den verwendeten Reaktoren zugespeist werden. Beispielsweise können ein paraffinhaltiger Stoffstrom und ein sauerstoffhaltiger Stoffstrom zu einem entsprechenden Reaktionseinsatzstrom in dem oder den verwendeten Reaktoren oder stromauf des oder der Reaktoren vereinigt werden.

Ein "Bilden" des Reaktionseinsatzstromes kann jegliche verfahrenstechnische Behandlung wie Verdichtung, Entspannung, Abkühlung oder Erwärmung oder auch die Abtrennung von Teilströmen, die Zuspeisung weiterer Stoffströme oder eine chemische Umsetzung von Komponenten umfassen. Insbesondere umfasst im Rahmen der vorliegenden Erfindung das Bilden des Reaktionseinsatzstromes beispielsweise, einen in den Reaktionseinsatzstrom überführten Stoffstrom zu erwärmen. Bei dieser Erwärmung, der sogenannten Feedvorwärmung, kann der Reaktionseinsatzstrom auf eine Temperatur gebracht werden, die die ODH in einer sich anschließenden Reaktionseinheit mit einem oder mehreren Reaktoren anlaufen lässt.

Insbesondere kann in einem Verfahren gemäß einer Ausführungsform der Erfindung vorgesehen sein, dass das Bilden des Reaktionseinsatzstromes umfasst, einen Stoffstrom mit einem oder mehreren weiteren Fluiden zu vereinigen. Auf diese Weise können geeignete Medien zugespeist werden, die beispielsweise die Reaktionsbedingungen bei der ODH günstig beeinflussen. Wie erwähnt, handelt es sich bei der ODH um eine stark exotherme Reaktion, so dass typischerweise sogenannte Verdünnungsmittel wie Inertgase oder Dampf zugegeben werden, um ein thermisches Durchgehen zu verhindern. Entsprechende Verdünnungsmittel können bei der Bildung des Reaktionseinsatzstromes zugegeben werden, wahlweise aber auch erst in einem oder mehreren Reaktoren. Auch kann beispielsweise bereits bei der Bildung des Reaktionseinsatzstromes Sauerstoff oder ein sauerstoffhaltiges Gasgemisch zugegeben werden, der bei der ODH benötigt wird. Wahlweise erfolgt auch dies erst später.

Im Rahmen der vorliegenden Erfindung wird vorteilhafterweise der Wasserpartialdruck gemessen und es wird eine Regelung verwendet, mittels derer der Wasserpartialdruck unter Verwendung wenigstens einer Stellgröße eingestellt wird. Wie erwähnt, kann durch eine Regelung auf Grundlage des Wasserpartialdrucks eine sehr viel genauere Einstellung des Produktverhältnisses erzielt werden, als wenn lediglich eine Wasserzugabe im Reaktionseinsatzstrom gesteuert würde.

Wie erwähnt kommt die vorliegende Erfindung insbesondere dann zum Einsatz, wenn in der oxidativen Dehydrierung ein Katalysator, der zumindest die Elemente Molybdän, Vanadium, Niob und optional Tellur enthält, also ein sogenannter MoVTeNbO-Katalysator, verwendet wird, weil sich bei Verwendung eines derartigen Katalysators Ethylen und Essigsäure bilden und die erwähnten Gesetzmäßigkeiten einstellen.

Die oxidative Dehydrierung wird im Rahmen der vorliegenden Erfindung vorteilhafterweise mit einer Ethankonversion von mindestens 15% durchgeführt. Die Ethankonversion kann dabei insbesondere bei mindestens 20, 25, 30, 35, 40 oder 45% liegen. Die Ethankonversion liegt insbesondere unterhalb von 75%. Das vorgegebene Produktmolenstromverhältnis von Essigsäure zu Ethylen liegt dabei insbesondere in einem Bereich von 0,05 bis 0,5.

Unter der "Konversion" bzw. dem "Umsatz" wird dabei hier der molare Anteil der eingesetzten Edukte, hier des Ethans, verstanden, der insgesamt zu (Haupt- und Neben-)Produkten reagiert. Der "Produktmolenstrom" einer Komponente beschreibt die Molmenge einer Komponente, die pro Zeiteinheit aus einem oder mehreren Reaktoren austritt.

Der Wasserpartialdruck in dem Prozessgas kann im Rahmen der vorliegenden Erfindung insbesondere durch Zugabe von Wasser zu dem Reaktionseinsatzstrom und/oder durch Einstellen einer Reaktortemperatur, bei der die oxidative Dehydrierung durchgeführt wird, eingestellt werden. Hierbei handelt es sich also um geeignete Stellgrößen der erwähnten Regelung. Es kann beispielsweise auch vorgesehen sein, mittels einer Wasserzugabe zu dem Reaktionseinsatzstrom eine Grob- und mittels der Einstellen einer Reaktortemperatur eine Feineinstellung vorzunehmen. Bei höherer Reaktortemperatur ergibt sich ein höherer Umsatz und damit eine höhere Bildung von Reaktionswasser. Hierbei wird der Wasserpartialdruck in dem Prozessgas also zumindest zum Teil durch Einstellen der Reaktortemperatur eingestellt. Als weitere maßgebliche Einflussgröße ergibt sich die zugegebene Menge an Sauerstoff im Reaktionseinsatzstrom. Im Rahmen der vorliegenden Erfindung wird dieser Parameter stets so angepasst, dass am Reaktoraustritt ein Sauerstoffgehalt im Prozessgas zwischen 0,01 mol-% und 50 mol-%, bevorzugt zwischen 0,1 und 5 mol-%, besonders bevorzugt zwischen 0,1 und 0,5 mol-% stets eingehalten wird, um einerseits eine Reduktion des Katalysatormaterials durch Sauerstoffmangel zu vermeiden und andererseits sicherheitstechnische Risiken aufgrund hoher Sauerstoffgehalte zu limitieren. Aus diesen Einschränkungen ergibt sich jedoch der Umstand, dass die Regelung der Sauerstoffzudosierung der grundlegenden Festlegung des Betriebspunktes nachgeschaltet ist und keinen nennenswerten Einfluss auf das Produktmolenstromverhältnis besitzt, solange sichergestellt ist, dass der oben genannte Bereich für den Sauerstoffgehalt am Austritt eingehalten wird.

Erfindungsgemäß wird als der einzustellende Wasserpartialdruck der Partialdruck an einem Reaktoraustritt eines oder mehrerer für die oxidative Dehydrierung verwendeter Reaktoren verstanden, beispielsweise direkt am Ende eines Katalysatorbetts oder einer mit diesem verbundenen Leitung. Insbesondere ist ein Prozessgas aus der oxidativen Dehydrierung am Reaktoraustritt noch keinen seine Zusammensetzung ändernden Maßnahmen, insbesondere einer Kühlung, einer Wäsche und dergleichen, unterworfen worden.

Der Wasserpartialdruck wird an dem Reaktoraustritt des oder der Reaktoren erfasst. Verfahren zur Wasserbestimmung und damit zur Bestimmung des Wasserpartialdrucks sind dem Fachmann grundsätzlich bekannt. Beispielsweise kann es sich hierbei um gängige Absorptionsspektroskopiemethoden, z.B. Fourier-transformierte Infrarotspektroskopie (FTIR) oder Tunable-Diode-Laser-Absorptionsspektroskopie (TDLAS), in Kombination mit gängigen Druckmessmethoden handeln.

Mit besonderem Vorteil wird die oxidative Dehydrierung im Rahmen der vorliegenden Erfindung in einem Temperaturbereich von 240 bis 500 °C in einem Reaktorbett des oder der verwendeten Reaktoren durchgeführt. Insbesondere kann der Temperaturbereich bei 260 und 400 °C, besonders bev orzugt bei 280 bis 350 °C liegen. Der Gesamtdruck am Reaktoreintritt des oder der Reaktoren liegt vorzugsweise zwischen 1 und 10 bar (abs.), insbesondere zwischen 2 und 9 bar (abs.), weiter insbesondere zwischen 3 und 8 bar (abs.). Die Raumgeschwindigkeit im Reaktorbett des oder der Reaktoren (WHSV) liegt im Bereich zwischen 0,1 und 10 kg Ethan/(h × kg Katalysator), bevorzugt zwischen 0,5 und 5 kg Ethan/(h × kg Katalysator), besonders bevorzugt zwischen 0,7 und 3 kg Ethan/(h × kg Katalysator). Insbesondere in diesem Bereich ist die zuvor erläuterte Einstellbarkeit der Produktmolenströme möglich.

Das erfindungsgemäße Verfahren kann insbesondere unter Verwendung eines oder mehrerer, dem Reaktionseinsatzstrom zugegebener und in das Prozessgas übergehender Verdünnungsmittel durchgeführt werden. Die Verwendung entsprechender Verdünnungsmittel, die insbesondere sicherstellen, dass bei der stark exothermen ODH ein stabiler und sicherer Reaktorbetrieb gewährleistet wird, ist grundsätzlich bekannt. Wie erwähnt, kann zur Einstellung des gewünschten Wasserpartialdrucks in dem genannten Bereich insbesondere eine Zugabe von Wasser bzw. Wasserdampf in den Reaktionseinsatzstrom erfolgen. Dieses Wasser bzw. dieser Wasserdampf wirkt zugleich als Verdünnungsmittel. Alternativ oder zusätzlich können aber ein oder mehrere weitere Verdünnungsmittel verwendet werden.

Insbesondere können im Rahmen der vorliegenden Erfindung ein oder mehrere Verdünnungsmittel zum Einsatz kommen, das oder die aus der Gruppe ausgewählt ist oder sind, die aus Wasser, Methan, Stickstoff und wenigstens einem weiteren Inertgas besteht. Auch Kohlendioxid kann als Verdünnungsmittel zum Einsatz kommen. Entsprechende Verdünnungsmittel nehmen an der Reaktion in dem oder den Reaktoren nicht oder allenfalls zu einem geringen Anteil teil und gehen daher zumindest zum überwiegenden Teil in das Prozessgas über.

Im Rahmen der vorliegenden Erfindung wurde ferner erkannt, dass auch bei Einbringung von Ethylen als zusätzlichem Feedstrom in den Reaktor, d.h. als Teil des Reaktionseinsatzstromes, ein starker funktionaler Zusammenhang zwischen dem Produktmolenstromverhältnis von Ethylen und Essigsäure und dem Wasserpartialdruck am Reaktoraustritt besteht. Die beschriebene Anlagenfahrweise kann somit auch bei zusätzlicher Ethyleneinspeisung angewandt werden. Dies ermöglicht es beispielsweise, die Flexibilität hin zu mehr Essigsäure als Produkt noch zu verstärken, bei allerdings dann zu erwartenden höheren Verlusten zu Kohlenmonoxid und Kohlendioxid. In bestimmten Fällen kann also eine Verfahrensvariante vorteilhaft sein, bei der dem Reaktionseinsatzstrom ferner Ethylen in einer vorgegebenen Menge, insbesondere von 0 bis 50 Molprozent, zugegeben wird.

Die Einbringung von zusätzlichem Ethylen kann sowohl in Form einer Einspeisung aus einer externen Quelle als auch in Form einer Rückführung einer entsprechenden Fraktion aus dem Zerlegungsteil der Anlage selbst erfolgen. Bei dem "Zerlegungsteil" handelt es sich um eine Anordnung, in der mittels thermischer Trennung Komponenten oder Komponentengruppen aus dem Prozessgas oder einem aus diesem erhaltenen Gasgemisch abgetrennt werden. Diese Rückführung kann durch zusätzliche Entnahme einer entsprechenden Fraktion im Zerlegungsteil oder durch Änderung der Sumpfproduktspezifikation in einer Rektifikationskolonne erfolgen, die zur Trennung von Ethan und Ethylen verwendet wird, und die im Zerlegungsteil vorgesehen ist. In diesem Fall wird durch Anpassung der Trennbedingungen wie Kopftemperatur oder Druck, oder aber durch Verwendung einer entsprechend ausgebildeten, "unschärfer" trennenden Rektifikationskolonne gezielt ein Teil des ansonsten über Kopf abgezogenen Produkts Ethylen in den Sumpf der Rektifikationskolonne überführt und dort in einer ansonsten überwiegend Ethan enthaltenden Fraktion abgezogen. Diese kann in den oder die Reaktoren zurückgeführt werden.

Die vorliegende Erfindung erstreckt sich ferner auf eine Anlage zur Herstellung von Ethylen und Essigsäure, die dafür eingerichtet ist, einen Ethan und Sauerstoff enthaltenden Reaktionseinsatzstrom zu bilden und einen Teil des Ethans und des Sauerstoffs in dem Reaktionseinsatzstrom unter Erhalt eines Prozessgases durch oxidative Dehydrierung zu dem Ethylen und der Essigsäure umzusetzen, wobei das Prozessgas den nicht umgesetzten Teil des Ethans und des Sauerstoffs, das Ethylen und die Essigsäure sowie Wasser enthält. Erfindungsgemäß ist vorgesehen, dass die Anlage Mittel aufweist, die dafür eingerichtet sind, in dem Prozessgas einen Wasserpartialdruck in Abhängigkeit von einem vorgegebenen Produktverhältnis von der Essigsäure zu dem Ethylen auf einen Wert in einem Bereich zwischen 0,5 und 5 bar (abs.), insbesondere in einem Bereich zwischen 0,7 und 3 bar (abs.) einzustellen.

Zu Merkmalen und Vorteilen einer entsprechenden Anlage sei auf die obigen Erläuterungen zu den Merkmalen und Vorteilen des Verfahrens verwiesen. Insbesondere ist eine derartige Anlage zur Durchführung eines Verfahrens gemäß den oben erläuterten spezifischen Ausgestaltungen eingerichtet und weist hierzu geeignete Mittel auf. Auch diesbezüglich sei auf die obigen Ausführungen verwiesen.

Die Erfindung wird nachfolgend unter Bezugnahme auf die beigefügten Zeichnungen näher erläutert, die unter anderem bevorzugte Ausführungsformen der vorliegenden Erfindung veranschaulichen.

### Kurze Beschreibung der Zeichnungen

Figur 1 veranschaulicht eine Anlage zur Herstellung von Ethylen und Essigsäure gemäß einer Ausführungsform der Erfindung.
Figur 2 veranschaulicht in einem Verfahren gemäß einer Ausführungsform der Erfindung erhaltene Selektivitäten zu Ethylen und Essigsäure.
Figur 3 veranschaulicht in einem Verfahren gemäß einer Ausführungsform der Erfindung erhaltene Produktmolenstromverhältnisse bezüglich Ethylen und Essigsäure.
Figur 4 veranschaulicht in einem Verfahren gemäß einer Ausführungsform der Erfindung erhaltene Produktmolenstromverhältnisse bezüglich Ethylen und Essigsäure.
Figur 5 veranschaulicht ein Verfahren gemäß einer Ausführungsform der Erfindung.
Figur 6 veranschaulicht Experimentdaten gemäß einer Ausführungsform der Erfindung.

### Ausführliche Beschreibung der Zeichnungen

In den nachfolgenden Figuren sind einander funktionell oder baulich entsprechende Elemente mit identischen Bezugszeichen angegeben und werden der Übersichtlichkeit halber nicht wiederholt erläutert. Werden nachfolgend Anlagenteile beschrieben, gelten die Erläuterungen zu diesen sinngemäß auch für die mittels dieser Anlagenteile implementierten Verfahrensschritte und umgekehrt.

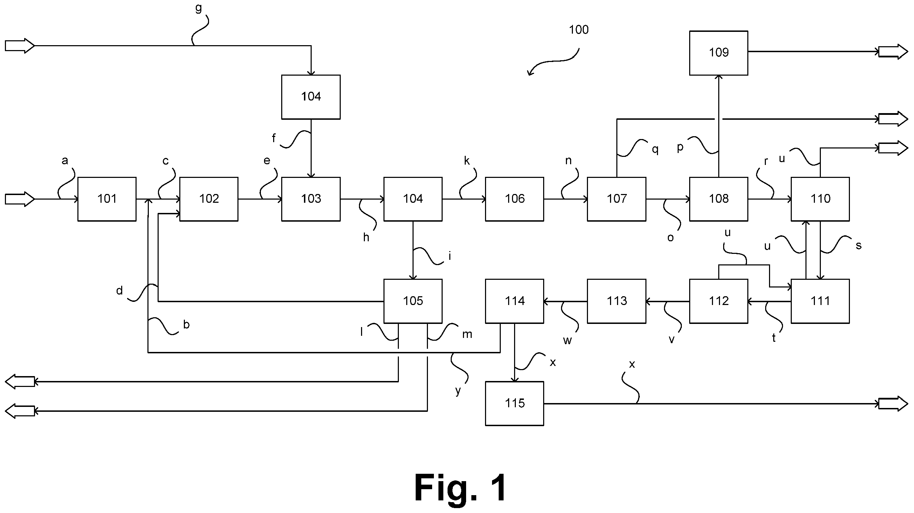

In Figur 1 ist eine Anlage zur Herstellung von Olefinen gemäß einer Ausführungsform der Erfindung in Form eines stark vereinfachten Anlagendiagramms veranschaulicht und insgesamt mit 100 bezeichnet. Wenngleich nachfolgend eine Anlage 100 zur ODH von Ethan (ODH-E) beschrieben wird, eignet sich, wie erwähnt, die vorliegende Erfindung auch zum Einsatz bei der ODH höherer Kohlenwasserstoffe. In diesem Fall gelten die nachfolgenden Erläuterungen entsprechend.

In der Anlage 100 wird einer Rektifikationseinheit 101 mit beispielsweise einer oder mehreren Rektifikationskolonnen ein Trenneinsatz in Form eines Stoffstroms a zugeführt und einer Rektifikation unterworfen. Der Trenneinsatz enthält im dargestellten Beispiel zumindest Ethan und höhere Kohlenwasserstoffe, insbesondere entsprechende höhere Paraffine. Der Rektifikationseinheit 101 können auch ein oder mehrere weitere Trenneinsätze zugeführt werden, beispielsweise der hier gezeigte und unten näher erläuterte Stoffstrom b.

In der Rektifikationseinheit 101 wird der Trenneinsatz alleine oder zusammen mit dem oder den weiteren Trenneinsätzen unter Erhalt eines Gasgemischs, das Ethan enthält, aber arm an höheren Kohlenwasserstoffen ist, einer Rektifikation unterworfen. Das Gasgemisch wird in Form eines Stoffstroms c abgezogen und einer Vorwärmeinheit 102 zugeführt. In der Vorwärmeinheit 102 wird das Gasgemisch vorgewärmt, wobei der Vorwärmeinheit 102 im dargestellten Beispiel auch ein Wasser- oder Dampfstrom d zugeführt wird. Auch weitere Stoffströme können zugeführt werden, wie hier in Form eines Stoffstroms b veranschaulicht. In der Rektifikationseinheit 101 wird ferner ein Komponentengemisch erhalten, das überwiegend oder ausschließlich die höheren Kohlenwasserstoffe enthält. Dieses ist nicht explizit gezeigt.

Ein aus der Vorwärmeinheit 102 abströmender Stoffstrom e wird zur Bildung eines Reaktionseinsatzstromes einer Reaktionseinheit 103 zugeführt. Der Reaktionseinsatzstrom enthält aufgrund seiner Bildung unter Verwendung des Trennprodukts aus der Rektifikationseinheit 101 Ethan, ist aber arm an höheren Kohlenwasserstoffen. Der Reaktionseinsatzstrom kann ferner ein oder mehrere Verdünnungsmittel wie Wasser oder Inertgase und weitere Komponenten enthalten. Diese können der Reaktionseinheit 103 auch in Form weiterer, nicht dargestellter Stoffströme zugeführt werden.

Der Reaktionseinheit 103 wird im dargestellten Beispiel ein sauerstoffhaltiger Stoffstrom f zugeführt. Dieser kann unter Verwendung einer Luftzerlegungsanlage 104 bereitgestellt werden. Der Luftzerlegungsanlage 104 wird hierzu ein Luftstrom g zugeführt. Bei dem sauerstoffhaltigen Stoffstrom f kann es sich um im Wesentlichen reinen Sauerstoff handeln, je nach Betrieb der Luftzerlegungsanlage 104 können jedoch auch Anteile an Stickstoff und an Edelgasen enthalten sein. Auf diese Weise ist es ebenfalls möglich, Verdünnungsmittel zuzuführen.

Aus der Reaktionseinheit 103 strömt ein Prozessgas in Form eines Prozessgasstroms h ab, in dem Ethylen enthalten ist, das in der Reaktionseinheit 103 durch ODH eines Teils des Ethans in dem Reaktionseinsatzstrom gebildet wurden. Ferner enthält das Produktgemisch Essigsäure, die ebenfalls bei der ODH in der Reaktionseinheit 103 aus Ethan gebildet wurde, Wasser, Kohlenmonoxid, Kohlendioxid, nicht umgesetzten Sauerstoff, sowie das oder die Verdünnungsmittel und weitere Verbindungen, falls zugegeben oder zuvor in der Reaktionseinheit 103 gebildet.

Es versteht sich, dass Reaktionseinheit 103 einen, aber auch mehrere, beispielsweise parallel betriebene, Reaktoren aufweisen kann. In letzterem Fall werden diesen Reaktoren jeweils entsprechende Reaktionseinsätze, die gleich oder unterschiedlich zusammengesetzt sein können, sowie entsprechende sauerstoffhaltige Stoffströme f zugeführt werden, und es werden jeweils entsprechende Prozessgasströme h gebildet. Letztere können beispielsweise vereinigt und gemeinsam als Prozessgas den nachfolgend erläuterten Einheiten zugeführt werden.

Stromab der Reaktionseinheit 103 kann ein Wasserpartialdruck erfasst werden. Dieser kann beispielsweise durch eine Zugabe von Wasser bzw. Dampf in Form des Stoffstroms d in die Vorwärmeinheit 102 eingestellt werden. Eine weitere Beeinflussung, insbesondere eine Feineinstellung, kann durch Einstellen der Temperatur in der Reaktionseinheit 103 erfolgen.

Das Prozessgas wird in eine Quencheinheit 104 überführt, in der es, beispielsweise in einer Waschsäule, mit Waschwasser oder einer geeigneten wässrigen Lösung in Kontakt gebracht werden kann. In der Quencheinheit 104 wird das Prozessgas insbesondere abgekühlt und die in der Reaktionseinheit 103 gebildete Essigsäure wird aus dem Prozessgas ausgewaschen. Mit Essigsäure beladenes Prozesswasser strömt in Form eines Stoffstroms i aus der Quencheinheit 104 ab, das zumindest weitgehend von Essigsäure befreite Prozessgas in Form eines Stoffstroms k.

In einer optionalen Essigsäurerückgewinnungseinheit 105 wird aus dem mit Essigsäure beladenen Prozesswasser Essigsäure als Eisessig abgetrennt, welcher als Stoffstrom I aus der Anlage 100 ausgeführt wird. In der Essigsäurerückgewinnungseinheit 105 ebenfalls rückgewonnenes reines Prozesswasser kann in Form des bereits erläuterten Stoffstroms d der Vorwärmeinheit 102 zugeführt werden. Das dem Reaktor zugeführte Prozesswasser kann auch teilweise oder ganz durch extern zugeführtes Frischwasser bereitgestellt werden. Nicht mehr brauchbares oder benötigtes Wasser kann in Form eines Abwasserstroms m aus der Anlage 100 ausgeführt und einer Abwasseraufbereitung zugeführt werden.

Das in Form des Stoffstroms k vorliegende, zumindest weitgehend von Essigsäure befreite Prozessgas wird in einer Verdichtungseinheit 106 auf ein geeignetes Druckniveau, beispielsweise 15 bis 25 bar, verdichtet und in Form eines verdichteten Stoffstroms n einer Aminwäscheeinheit 107 zugeführt. In dieser werden insbesondere Teile des in dem Prozessgas enthaltenen Kohlendioxids ausgewaschen. Nach Regenerierung des Amins kann das ausgewaschene Kohlendioxid in Form eines Stoffstroms q aus der Anlage ausgeführt werden. Das derart teilweise von Kohlendioxid befreite Prozessgas wird in Form eines Stoffstroms o in eine Laugenwäscheeinheit 108 überführt und dort weiter von Kohlendioxid gereinigt. In der Laugenwäscheeinheit 108 fällt Ablauge an, die in Form eines Stoffstroms p in eine Ablaugebehandlungseinheit 109 überführt und schließlich aus der Anlage 100 ausgeführt werden kann.

Das in der Laugenwäscheeinheit 108 weiter gereinigte Prozessgas wird in Form eines Stoffstroms r in eine Vorkühl- und Trockeneinheit 110 überführt, wo es insbesondere von Restwasser befreit werden kann. Das getrocknete Prozessgas wird in Form eines Stoffstroms s in eine Tieftemperatureinheit 111 überführt und anschließend in weiter abgekühlter Form in Form eines oder mehrerer Stoffströme t in eine Demethanisierungseinheit 112. In der Tieftemperatureinheit 111 und der Demethanisierungseinheit 112 werden tiefer als Ethylen siedende Komponenten, darunter insbesondere Kohlenmonoxid und Sauerstoff, aus dem Prozessgas abgetrennt, wobei der Rest in kondensierter Form verbleibt. Sind in dem Prozessgas höhere Kohlenwasserstoffe enthalten, die bei der ODH in der Reaktionseinheit 103 als Nebenprodukt gebildet wurden, gehen diese ebenfalls in das Kondensat über.

Die abgetrennten, tiefer als Ethylen siedenden Komponenten werden in Form eines oder mehrerer Stoffströme u durch die Tieftemperatureinheit 111 und die Vorkühl- und Trockeneinheit 110 zurückgeführt, dort ggf. mit weiteren entsprechender Stoffströme vereinigt, zu Kühlzwecken genutzt, und aus der Anlage 100 ausgeführt. Die Kohlenwasserstoffe mit zwei und ggf. mehr Kohlenstoffatomen werden bei Bedarf in Form eines Stoffstroms v einer Hydriereinheit 113 zugeführt, in der insbesondere Acetylen, das ebenfalls bei der ODH in der in der Reaktionseinheit 103 als Nebenprodukt gebildet wurde, hydriert werden kann. Nach der Hydrierung wird der nun mit w bezeichnete Stoffstrom in eine Ethylenabtrenneinheit 114 überführt.

In der Ethylenabtrenneinheit 114 wird Ethylen zumindest weitgehend von anderen Komponenten abgetrennt und kann in Form eines Stoffstroms x nach Nutzung in einer Ethylenkälteeinheit 115 gasförmig aus der Anlage 100 ausgeführt werden. Die übrigen Komponenten, überwiegend Ethan und ggf. höhere Kohlenwasserstoffe, werden in Form eines Stoffstroms y abgezogen. Sind in diesem höhere Kohlenwasserstoffe enthalten, werden diese vorteilhafterweise in Form des bereits erwähnten Stoffstroms b in die Reaktionseinheit zurückgeführt. Es kann eine optionale Aufbereitung dieses Stoffstroms b erfolgen.

Durch einen angepassten Betrieb der Ethylenabtrenneinheit 114 bzw. eine entsprechende Auslegung kann ein Teil des Ethylens auch in den Stoffstrom y bzw. b überführt und in diesem in das Verfahren zurückgeführt werden. Es ist auch möglich, den Stoffstrom x oder einen Teil hiervon zurückzuführen. Auf diese Weise lässt sich, wie erwähnt, die Ausbeute an Essigsäure bei Bedarf erhöhen. Es sei ausdrücklich betont, dass im Rahmen der vorliegenden Erfindung auf bestimmte Anlagenteile, insbesondere beispielsweise die Rektifikationseinheit 101, verzichtet werden kann. In diesem Fall kann der Stoffstrom y beispielsweise auch direkt in die Vorwärmeinheit 102 oder in die Reaktionseinheit 103 eingespeist werden. Weitere Varianten sind möglich und von der vorliegenden Erfindung umfasst.

Figur 2 veranschaulicht in einem Verfahren gemäß einer Ausführungsform der Erfindung erhaltene Selektivitäten zu Ethylen und Essigsäure in einem Diagramm, in dem Wasserpartialdrücke in bar (abs.) auf der Abszisse gegen Selektivitätswerte in Prozent auf der Ordinate aufgetragen sind. Die gezeigten Selektivitätswerte zu den einzelnen Produkten errechnen sich aus dem Verhältnis des jeweiligen Produktmolenstroms bezogen auf die molare Menge an Ethan, die pro Zeiteinheit im Reaktor umgesetzt wird.

Die gezeigten Daten beziehen sich auf zwei Versuchsreihen mit unterschiedlichen Durchströmraten, somit unterschiedlichen Raumgeschwindigkeiten und unterschiedlichen Temperaturen. Bei beiden Versuchsreihen wurde kein Ethylen am Reaktoreintritt hinzugegeben. Bei höheren Durchströmraten zeigen sich erwartungsgemäß niedrigere Umsätze (ca. 19% gegenüber ca. 40%), jedoch sind die Produktselektivitäten und somit das Produktmolenstromverhältnis (entspricht hier dem Verhältnis der beiden Selektivitäten) bei gleichen Wasserpartialdrücken am Reaktoraustritt nahezu identisch. Dies zeigt, dass die Prozesssteuerung in dem genannten Bereich maßgeblich auf den Wasserpartialdruck am Austritt gestützt werden kann.

Die bei den höheren Durchström- und geringeren Konversionsraten erhaltenen Werte sind für Ethylen mit ausgefüllten (schwarzen) Quadraten und für Essigsäure mit ausgefüllten (schwarzen) Dreiecken veranschaulicht, die bei den geringeren Durchström- und höheren Konversionsraten erhaltenen Werte entsprechend für Ethylen mit nicht ausgefüllten (weißen) Quadraten und für Essigsäure mit nicht ausgefüllten (weißen) Dreiecken.

Das Verhältnis der Produktmengen als Funktion des Wasserpartialdrucks am Reaktoraustritt ist nochmals in Figur 3 veranschaulicht. Hier sind die Wasserpartialdrücke in bar (abs.) auf der Abszisse gegen das Produktmolenstromverhältnis von Essigsäure zu Ethylen (entspricht hier dem Verhältnis der in Figur 2 gezeigten Werte zueinander) aufgetragen. Hier sind die Produktmolenstromverhältnisse für die höheren Durchström- und geringeren Konversionsraten mit ausgefüllten (schwarzen) Quadraten und für die geringeren Durchström- und höheren Konversionsraten mit nicht ausgefüllten (weißen) Quadraten veranschaulicht. Der teilweise klar lineare Verlauf des Produktmix wird vor allem für den wirtschaftlich relevanten Betrieb bei höheren Konversionen ersichtlich.

Dieses vereinfachte Verhalten des Reaktionssystems kann durch zwei Effekte erklärt werden, die experimentell nachgewiesen werden konnten, aber hier explizit als unverbindlich angegeben werden: Zum einen wird bei erhöhten Wasserpartialdrücken die Oxidation von gebildetem Ethylen begünstigt, wobei die Selektivität zur Bildung von Essigsäure zunimmt. Gleichzeitig wird die Desorption der gebildeten Essigsäure von der Katalysatoroberfläche durch erhöhte Wasserpartialdrücke begünstigt, wodurch weniger Essigsäure der ebenfalls am Katalysator stattfindenden nachfolgenden Oxidation von Essigsäure zu Kohlenmonoxid und Kohlendioxid zur Verfügung steht. Daher ergibt sich die Verschiebung der Gesamtselektivität hin zur Essigsäure, bei nahezu gleichbleibender Selektivität zu Kohlenmonoxid und Kohlendioxid.

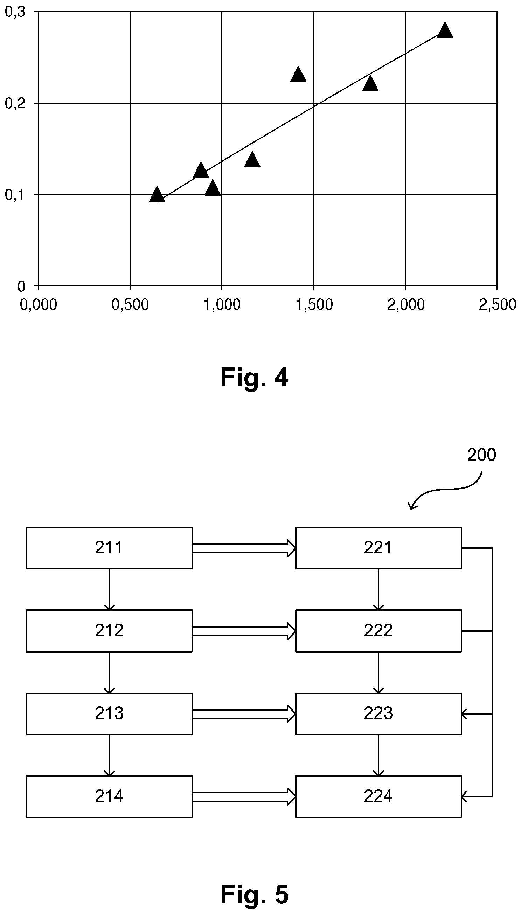

Der bestimmende Einfluss des Wasserpartialdrucks am Austritt auf das Produktverhältnis zwischen Essigsäure und Ethylen kann durch weitere Messungen belegt werden, zum Teil unter Verwendung unterschiedlicher Verdünnungsmedien und stark variierender Versuchsbedingungen. Hierzu wird auf Figur 4 verwiesen, die entsprechende Produktmolenstromverhältnisse von Essigsäure zu Ethylen zeigt. Die Darstellung entspricht jener der Figur 3.

Figur 5 veranschaulicht ein Verfahren gemäß einer Ausführungsform der Erfindung in Form eines schematischen Ablaufplans, der insgesamt mit 200 bezeichnet ist. Mit 211 bis 214 sind jeweils zu erreichende Teilziele, mit 221 bis 224 die hierzu konkret vorzunehmenden Einstellungen bzw. Vorgaben bezeichnet.

Die gewünschte Produktverteilung von Essigsäure zu Ethylen wird in Schritt 211 vorgegeben. Auf dieser Grundlage wird in Schritt 221 ein Zielwert für den Wasserpartialdruck am Reaktoraustritt festgelegt. Auf Grundlage einer in Schritt 212 vorgegebenen Gesamtproduktmenge und zugehöriger Recyclemengen wird in Schritt 222 eine Durchflussmenge und damit die Konversion im Reaktor (siehe hierzu insbesondere Figuren 2 und 3) festgelegt.

In Schritt 213 wird ein entsprechend definierter Betriebspunkt angefahren, wozu in Schritt 223 ein Wasseranteil im Reaktionseinsatzstrom eingestellt wird. Die Feinabstimmung des Betriebspunkts, Schritt 214, erfolgt durch eine Anpassung der Reaktortemperatur in Schritt 224. Es wird jeweils der Wasserpartialdruck am Reaktoraustritt beobachtet.

In Figur 6 sind die Ergebnisse dreier ausgewählter Experimente 52, 56 und 71 veranschaulicht, die im Rahmen einer umfangreichen Versuchsreihe unter Verwendung eines Pilotreaktors durchgeführt wurden. Wiederum wurde im Rahmen der gesamten Versuchsreihe eine starke Korrelation des Produktverhältnisses von Ethylen zu Essigsäure zum Wasserpartialdruck am Austritt des Reaktors beobachtet. Dies gilt für unterschiedliche Konversionen und unterschiedliche Prozessbedingungen, d.h. veränderte Zusammensetzungen, Strommengen, Drücke und Temperaturen.

Die Experimente 52 und 71 wurden dabei bei gleichen Raumgeschwindigkeiten von 0,9 kg Ethan/(kg Katalysator × h) durchgeführt; im Experiment 56 betrug diese hingegen 1,4 kg Ethan/(kg Katalysator × h). Die Wasserpartialdrücke am Reaktoreintritt lagen für Experiment 52 bei 0,56 bar, für Experiment 56 bei 0,58 bar und für Experiment 71 bei 0,46 bar. Mit anderen Worten wurden in den Experimenten 52 und 56 nahezu identische Wasserpartialdrücke am Reaktoreintritt verwendet und bei Experiment 71 wich der Wasserpartialdruck am Reaktoreintritt deutlich ab. Die Wasserpartialdrücke am Reaktoraustritt lagen für Experiment 52 bei 1,28 bar, für Experiment 56 bei 0,99 bar und für Experiment 71 bei 1,00 bar. Mit anderen Worten wurden also in den Experimenten 56 und 71 nahezu identische Wasserpartialdrücke am Reaktoraustritt beobachtet und bei Experiment 52 wich der Wasserpartialdruck am Reaktoraustritt deutlich ab. Die unterschiedlichen Wasserpartialdrücke am Reaktoraustritt zwischen den Experimenten 52 und 56 ergaben sich aus den unterschiedlichen Raumgeschwindigkeiten bei im Wesentlichen gleichen Wasserpartialdrücken am Reaktoreintritt.

Die Versuchsbedingungen für die Experimente 52, 56 und 71 sind in der nachfolgenden Tabelle nochmals zusammengefasst. Die Salztemperatur stellt dabei die Temperatur einer Salzschmelze dar, die zur Kühlung des Reaktors verwendet wurde und bildet daher ein Maß für die Reaktortemperatur:

| **Experiment Nr.** | **52** | **56** | **71** |
|---|---|---|---|
| Reaktoreintrittsdruck [bar (abs.)] | 3,81 | 3,67 | 3,10 |
| Raumgeschwindigkeit [kg Ethan/(kg Katalysator × h)] | 0,9 | 1,4 | 0,9 |
| Wasser/Ethan [mol/mol] | 0,26 | | |
| Sauerstoff/Ethan [mol/mol] | 0,35 | 0,31 | 0,33 |
| Salztemperatur [°C] | 302 | 316 | 311 |
| Wasserpartialdruck Reaktoreintritt [bar (abs.)] | 0,56 | 0,58 | 0,46 |
| Wasserpartialdruck Reaktoraustritt [bar (abs.)] | 1,28 | 0,99 | 1,00 |

In Experiment 52 wurde ein Einsatz mit 56,7 Molprozent Ethan, 19,6 Molprozent Sauerstoff, 14,8 Molprozent Wasser und 8,9 Molprozent Stickstoff, in Experiment 56 ein Einsatz mit 60,2 Molprozent Ethan, 18,4 Molprozent Sauerstoff, 15,8 Molprozent Wasser und 5,7 Molprozent Stickstoff und in Experiment 71 wurde ein Einsatz mit 57,3 Molprozent Ethan, 18,8 Molprozent Sauerstoff, 14,9 Molprozent Wasser und 9,0 Molprozent Stickstoff verwendet.

In Figur 6 sind Werte zur Selektivität (S) für Ethylen (C2H4), Essigsäure (AcOH), Kohlenmonoxid (CO), Kohlendioxid (CO2) und restliche Verbindungen (Rest, aufgrund geringer Werte nicht sichtbar) für die drei Experimente 52, 56 und 71 veranschaulicht.

Die Ordinate zeigt dabei die Werte bezüglich der Selektivitäten. Der Ethanumsatz variierte in den drei Experimenten 52, 56 und 71 um nicht mehr als 5%

Es zeigt sich deutlich, dass bei den Experimenten 56 und 71 ähnliche Produktverhältnisse bei ähnlichen Wasserpartialdrücken am Austritt bei unterschiedlichen Wasserpartialdrücken am Eintritt zu beobachten sind. Das Produktmolenstromverhältnis von Essigsäure zu Ethylen (entspricht hier dem Verhältnis der entsprechenden Selektivitäten) beträgt bei den Experimenten 56 und 71 jeweils rund 0,14. In den Experimenten 52 und 56 liegen hingegen ähnliche Wasserpartialdrücke am Eintritt, jedoch aufgrund der veränderten Raumgeschwindigkeiten deutlich unterschiedliche Wasserpartialdrücke am Austritt vor. Trotz ähnlicher Wasserpartialdrücke am Eintritt ergeben sich für die Versuchspunkte 52 und 56 auch deutlich unterschiedliche Produktverhältnisse. Das Produktmolenstromverhältnis von Essigsäure zu Ethylen beträgt rund 0,17 für Experiment 52 und liegt somit deutlich oberhalb des oben genannten Wertes für Experiment 56.

## Patentansprüche

1. Verfahren zur Herstellung von Ethylen und Essigsäure, bei dem ein Ethan und Sauerstoff enthaltender Reaktionseinsatzstrom gebildet und ein Teil des Ethans und des Sauerstoffs in dem Reaktionseinsatzstrom unter Erhalt eines Prozessgases durch oxidative Dehydrierung zu dem Ethylen und der Essigsäure umgesetzt wird, wobei das Prozessgas den nicht umgesetzten Teil des Ethans und des Sauerstoffs, das Ethylen und die Essigsäure sowie Wasser enthält, **dadurch gekennzeichnet, dass** das Verfahren umfasst, in dem Prozessgas einen Wasserpartialdruck in Abhängigkeit von einem vorgegebenen Produktverhältnis von der Essigsäure zu dem Ethylen auf einen Wert in einem Bereich zwischen 0,5 und 5 bar (abs.), insbesondere zwischen 0,7 und 3 bar (abs.), einzustellen, wobei für die oxidative Dehydrierung ein oder mehrere Reaktoren verwendet werden und der Wasserpartialdruck an einem Reaktoraustritt des oder der Reaktoren in dem Bereich eingestellt wird.

2. Verfahren nach Anspruch 1, bei dem der Wasserpartialdruck gemessen wird und bei dem eine Regelung verwendet wird, mittels derer der Wasserpartialdruck unter Verwendung wenigstens einer Stellgröße eingestellt wird.

3. Verfahren nach Anspruch 1 oder 2, bei dem der Wasserpartialdruck in dem Prozessgas durch Zugabe von Wasser zu dem Reaktionseinsatzstrom und/oder durch Einstellen einer Reaktortemperatur eingestellt wird.

4. Verfahren nach einem der vorstehenden Ansprüche, bei dem in der oxidativen Dehydrierung ein Katalysator verwendet wird, der zumindest die Elemente Molybdän, Vanadium, Niob und optional Tellur enthält.

5. Verfahren nach einem der vorstehenden Ansprüche, bei dem der Ethanumsatz bei der oxidativen Dehydrierung bei über 15% liegt.

6. Verfahren nach einem der vorstehenden Ansprüche, bei dem der Wasserpartialdruck an dem Reaktoraustritt des oder der Reaktoren erfasst wird.

7. Verfahren nach einem der vorstehenden Ansprüche, bei dem die oxidative Dehydrierung in einem Temperaturbereich von 240 bis 500°C in einem Reaktorbett des oder der Reaktoren durchgeführt wird.

8. Verfahren nach einem der vorstehenden Ansprüche, bei dem eine Raumgeschwindigkeit in dem Reaktorbett des oder der Reaktoren in einem Bereich zwischen 0,1 und 10 kg Ethan/(h × kg Katalysator) liegt.

9. Verfahren nach einem der vorstehenden Ansprüche, bei dem ein Gesamtdruck an einem Reaktoreintritt des oder der Reaktoren zwischen 1 und 10 bar (abs.) liegt.

10. Verfahren nach einem der vorstehenden Ansprüche, bei dem dem Reaktionseinsatzstrom ferner ein oder mehrere, in das Prozessgas übergehende Verdünnungsmittel zugegeben werden.

11. Verfahren nach Anspruch 10, bei dem das oder die Verdünnungsmittel aus der Gruppe ausgewählt ist oder sind, die aus Wasser, Methan, Stickstoff, Kohlendioxid und wenigstens einem weiteren Inertgas besteht.

12. Verfahren nach einem der vorstehenden Ansprüche, bei dem dem Reaktionseinsatzstrom ferner Ethylen zugegeben wird.

13. Verfahren nach einem der vorstehenden Ansprüche, bei dem dem Reaktionseinsatzstrom ein zumindest einen Teil des Ethylens sowie zumindest einen Teil des Ethans enthaltendes Einsatzgemisch zugegeben wird.

14. Anlage (100) zur Herstellung von Ethylen und Essigsäure, die dafür eingerichtet ist, einen Ethan und Sauerstoff enthaltenden Reaktionseinsatzstrom zu bilden und einen Teil des Ethans und des Sauerstoffs in dem Reaktionseinsatzstrom unter Erhalt eines Prozessgases durch oxidative Dehydrierung zu dem Ethylen und der Essigsäure umzusetzen, wobei das Prozessgas den nicht umgesetzten Teil des Ethans und des Sauerstoffs, das Ethylen und die Essigsäure sowie Wasser enthält, **dadurch gekennzeichnet, dass** die Anlage (100) Mittel aufweist, die dafür eingerichtet sind, in dem Prozessgas einen Wasserpartialdruck in Abhängigkeit von einem vorgegebenen Produktverhältnis von der Essigsäure zu dem Ethylen auf einen Wert in einem Bereich zwischen 0,5 und 5 bar (abs.), insbesondere zwischen 0,7 und 3 bar (abs.), einzustellen, wobei für die oxidative Dehydrierung ein oder mehrere Reaktoren bereitgestellt sind und Mittel bereitgestellt sind, den Wasserpartialdruck an einem Reaktoraustritt des oder der Reaktoren in dem Bereich einzustellen.

## Claims

1. Method for the production of ethylene and acetic acid, wherein a reaction charge stream containing ethane and oxygen is formed, and a portion of the ethane and the oxygen in the reaction charge stream is converted into ethylene and acetic acid via oxidative dehydrogenation to obtain a process gas, wherein the process gas contains the unconverted portion of the ethane and the oxygen, the ethylene, and the acetic acid as well as water, **characterized in that** the method includes setting a water partial pressure to a value in a range of between 0.5 and 5 bar (abs.), in particular between 0.7 and 3 bar (abs.), in the process gas as a function of a predetermined product ratio of the acetic acid to the ethylene, wherein one or more reactors are used for the oxidative dehydrogenation, and the water partial pressure is set in this range at a reactor outlet of the reactor or reactors.

2. The method according to claim 1, wherein the water partial pressure is measured, and in which a control is used by means of which the water partial pressure is adjusted by using at least one control variable.

3. The method according to claim 1 or 2, wherein the water partial pressure in the process gas is adjusted by adding water to the reaction charge stream and/or by adjusting a reactor temperature.

4. The method according to any of the preceding claims, wherein a catalyst which contains at least the elements molybdenum, vanadium, niobium, and optionally tellurium is used in the oxidative dehydrogenation.

5. The method according to any of the preceding claims, wherein the ethane conversion in the oxidative dehydrogenation is above 15 %.

6. The method according to any of the preceding claims, wherein the water partial pressure is detected at the reactor outlet of the reactor or reactors.

7. The method according to any of the preceding claims, wherein the oxidative dehydrogenation is performed in a temperature range of from 240 to 500 °C in a reactor bed of the reactor or reactors.

8. The method according to any of the preceding claims, wherein a space velocity in the reactor bed of the reactor or reactors is in a range between 0.1 and 10 kg ethane/(h x kg catalyst).

9. The method according to any of the preceding claims, wherein a total pressure at a reactor inlet of the reactor or reactors is between 1 and 10 bar (abs.).

10. The method according to any of the preceding claims, wherein one or more diluents which pass into the process gas are further added to the reaction charge stream.

11. The method according to claim 10, wherein the diluent or diluents are selected from the group consisting of water, methane, nitrogen, carbon dioxide, and at least one further inert gas.

12. The method according to any of the preceding claims, wherein ethylene is also added to the reaction charge stream.

13. The method according to any of the preceding claims, wherein a charge mixture containing at least a portion of the ethylene and at least a portion of the ethane is added to the reaction charge stream.

14. Installation (100) for the production of ethylene and acetic acid, which is configured to form a reaction charge stream containing ethane and oxygen and to convert a portion of the ethane and the oxygen in the reaction charge stream into ethylene and acetic acid via oxidative dehydrogenation to obtain a process gas, wherein the process gas contains the unconverted portion of the ethane and the oxygen, the ethylene, and the acetic acid as well as water, **characterized in that** the installation (100) comprises means that are configured to set a water partial pressure to a value in a range of between 0.5 and 5 bar (abs.), in particular between 0.7 and 3 bar (abs.), in the process gas as a function of a predetermined product ratio of acetic acid to ethylene, wherein one or more reactors are provided for the oxidative dehydrogenation, and means are provided for setting the water partial pressure in the range at a reactor outlet of the reactor or reactors.

## Revendications

1. Procédé de production d'éthylène et d'acide acétique, dans lequel un flux initial de réaction contenant de l'éthane et de l'oxygène est formé et une partie de l'éthane et de l'oxygène dans le flux initial de réaction est transformée en éthylène et acide acétique par déshydrogénation oxydative avec obtention d'un gaz de procédé, le gaz de procédé contenant la partie non transformée de l'éthane et de l'oxygène, l'éthylène et l'acide acétique ainsi que de l'eau, **caractérisé en ce que** le procédé comprend le réglage dans le gaz de procédé d'une pression partielle d'eau en fonction d'un rapport de produits défini au préalable de l'acide acétique à l'éthylène à une valeur située dans la plage comprise entre 0,5 et 5 bar (abs.), en particulier entre 0,7 et 3 bar (abs.), un ou plusieurs réacteurs étant utilisés pour la déshydrogénation oxydative et la pression partielle de l'eau à une sortie du ou des réacteurs étant réglée dans la plage.

2. Procédé selon la revendication 1, dans lequel la pression partielle de l'eau est mesurée et dans lequel un réglage est utilisé, au moyen duquel la pression partielle de l'eau est réglée en utilisant au moins une grandeur de réglage.

3. Procédé selon la revendication 1 ou 2, dans lequel la pression partielle de l'eau dans le gaz de procédé est réglée par l'ajout d'eau au flux initial de réaction et/ou par le réglage d'une température de réacteur.

4. Procédé selon l'une quelconque des revendications précédentes, dans lequel la déshydrogénation oxydative utilise un catalyseur, qui contient au moins les éléments molybdène, vanadium, niobium et facultativement tellure.

5. Procédé selon l'une quelconque des revendications précédentes, dans lequel la conversion de l'éthane par la déshydrogénation oxydative est supérieure à 15 %.

6. Procédé selon l'une quelconque des revendications précédentes, dans lequel la pression partielle de l'eau est mesurée à la sortie du ou des réacteurs.

7. Procédé selon l'une quelconque des revendications précédentes, dans lequel la déshydrogénation oxydative est réalisée dans une plage de températures allant de 240 à 500 °C dans un lit du ou des réacteurs.

8. Procédé selon l'une quelconque des revendications précédentes, dans lequel une vitesse spatiale dans le lit du ou des réacteurs se situe dans une plage comprise entre 0,1 et 10 kg d'éthane/(h x kg de catalyseur).

9. Procédé selon l'une quelconque des revendications précédentes, dans lequel une pression totale à une entrée du ou des réacteurs est comprise entre 1 et 10 bar (abs.).

10. Procédé selon l'une quelconque des revendications précédentes, dans lequel un ou plusieurs diluants circulant dans le gaz de procédé sont également ajoutés au flux initial de réaction.

11. Procédé selon la revendication 10, dans lequel le ou les diluants sont choisis parmi le groupe constitué par l'eau, le méthane, l'azote, le dioxyde de carbone et au moins un autre gaz inerte.

12. Procédé selon l'une quelconque des revendications précédentes, dans lequel de l'éthylène est également ajouté au flux initial de réaction.

13. Procédé selon l'une quelconque des revendications précédentes, dans lequel un mélange de charge contenant au moins une fraction de l'éthylène ainsi qu'au moins une fraction de l'éthane est ajouté au flux initial de réaction.

14. Installation (100) de production d'éthylène et d'acide acétique, conçue pour former un flux initial de réaction contenant de l'éthane et de l'oxygène et pour transformer une partie de l'éthane et de l'oxygène dans le flux initial de réaction en éthylène et acide acétique par déshydrogénation oxydative avec obtention d'un gaz de procédé, le gaz de procédé contenant la partie non transformée de l'éthane et de l'oxygène, l'éthylène et l'acide acétique ainsi que de l'eau, **caractérisée en ce que** l'installation (100) comporte des moyens qui sont conçus pour régler dans le gaz de procédé une pression partielle d'eau en fonction d'un rapport de produits défini au préalable de l'acide acétique à l'éthylène à une valeur située dans une plage comprise entre 0,5 et 5 bar (abs.), en particulier entre 0,7 et 3 bar (abs.), un ou plusieurs réacteurs étant fournis pour la déshydratation oxydative et des moyens étant fournis pour régler la pression partielle d'eau à une sortie du ou des réacteurs dans la plage.
